# EUROPEAN PATENT APPLICATION

(11) **EP 2 163 214 A1**
(43) Date of publication of application: **17.03.2010**
(21) Application number: 09010372.2
(22) Date of filing: 12.08.2009
(51) Int. Cl.: A61B 17/34, A61M 1/00, A61M 27/00

(54) **Structure for connecting between a medical needle and medical tubing**

(30) Priority: 12.09.2008 JP 2008234272
(71) Applicant: Tyco Healthcare Group LP, Mansfield, MA 02048 (US)
(72) Inventor: Sakai, Yosuke, Fukuroi Shizuoka 437-0004 (JP); Tsukanaka, Aya, Fukuroi Shizuoka 437-0004 (JP); Satoh, Takashi, Fukuroi Shizuoka 437-0004 (JP); Atsumi, Taiki, Fukuroi Shizuoka 437-0004 (JP)
(74) Representative: Olsson, Carl H.S.

(57) **Abstract**

[Problem]

To provide a structure for connecting between a medical needle and medical tubing wherein no unevenness occurs in the connecting portion between the medical needle and medical tubing, and moreover its cost is low.

[Means of Solving]

Connecting structure A comprises a medical needle 10 with a connector 13 provided with a joining groove 13a formed on its rear-end portion, an inner tube 16 that joins to the joining groove 13a, and medical tubing 15 that has one end secured to the outer peripheral surface of the inner tube 16 by welding or adhesive. Moreover, the connector 13 and the inner tube 16 are shaped so as to form a cylinder when the inner tube 16 is fit to the joining groove 13a of the connector 13. In addition, a portion that is not covered by the inner tube 16 is provided at the base-end portion of the connector 13, thus providing a gap between the base-end portion of the connector 13 and the medical tubing 15.

## Description

### [Technical Field]

The present invention relates to the structure of a connection between a medical needle and medical tubing that is left in a wound after surgery.

### [Background Art]

Conventionally, after surgery, medical tubing is inserted into a wound to perform the drainage of blood or other exudates that may arise in the wound. In such a case, a medical needle is attached to the tip of the medical tubing, and the medical needle is passed from the wound toward the outside of the body of the patient, thus leaving the rear-end portion of the medical tubing in the wound. Moreover, after removing the medical needle from the tip of the medical tubing, the tip of a tube extending from a storage container via a pump is connected to this tip and the pump is operated to suck the blood or other exudates from the wound into the interior of the storage container (see, for example, Patent Reference 1).

This wound drain catheter (medical tubing) is connected to a trocar (medical needle) via a drain tube constituting the tip portion, and a connector portion is formed at the rear end of the trocar where the drain tube is connected. This connector portion is provided with a plurality of annular protrusions, and these protrusions are joined in a grasping manner to the inside wall of the drain tube, and thus the wound drain catheter is connected to the trocar. [Patent Reference 1] Japanese patent application publication number JP H2-17185 A

### [Disclosure of the Invention]

However, the aforementioned conventional wound drain catheter is connected in a connection portion where a plurality of annular protrusions is formed, so the shape of the steps in the protrusions is exposed on the outside surface of the wound drain catheter, and so the surface of the wound drain catheter is uneven. For this reason, greater resistance arises when piercing the wound drain catheter into the body of the patient, so smooth handling becomes more difficult. In addition, while method whereby protrusions with steps are not used, but rather adhesive or the like is used for connecting to the medical tubing at a cylindrical connecting portion is conceivable, with this method it is difficult to maintain adequate adhesion, and there is a problem in that additional costs would be required to achieve this adhesion.

In consideration of these circumstances, the present invention has as its object to provide a structure for connecting between a medical needle and medical tubing wherein no unevenness occurs in the connecting portion between the medical needle and medical tubing, and moreover its cost is low.

In order to achieve the aforementioned object, the structure for connecting between a medical needle and medical tubing according to the present invention is characterized in comprising: a medical needle wherein a connector provided with a joint is formed upon its rear end; a conjugating member that is joined to said joint in the state such that it covers at least part of said connector, and is formed in a cylindrical shape at said connector when it covers at least part of said connector; and medical tubing that is connected to said medical needle by covering a cylindrical body made up of said connector and said conjugating member in the state in which the inside surface at one end is secured by deposition or adhesion to the outside surface of said conjugating member.

The structure for connecting between a medical needle and medical tubing according to the present invention does not provide unevenness at the connector to the medical needle, thus joining the medical tubing at the uneven connector, but rather at least part of the connector is covered with a conjugating member that is able to join to the connector, thus causing the union between the connector and the conjugating member to be formed in a cylindrical shape. Moreover, one end of the medical tubing is secured to the outside surface of the cylindrically formed connector and conjugating member. In this case, the conjugating member and medical tubing are secured by welding or adhesion. To wit, the material to constitute the conjugating member is selected such that it can be secured to the medical tubing by welding or adhesion. Thereby, there is no unevenness on the outside surface of the union between the connector and conjugating member located in the interior of the medical tubing, so no unevenness occurs on the surface of the medical tubing either. As a result, the medical tubing exhibits little resistance when piercing the body of the patient, so smooth handling becomes possible.

In addition, the structure for connecting between a medical needle and medical tubing according to the present invention is further **characterized in that:** said connector is formed in a cylindrical shape; said joint is constituted with a groove formed on the outside surface of said connector along the circumference thereof; said conjugating member is constituted with an elastic cylindrical body that joins to said groove; and also, the thickness of said conjugating member is set to be approximately the same as the depth of said groove.

Thereby, it is possible to obtain a structure for connecting between a medical needle and medical tubing that has a simple constitution and wherein no unevenness occurs in the connecting portion. In addition, the conjugating member comprises a material that is elastic, so even if the conjugating member is made from a single cylindrical body, the conjugating member can be joined to the groove by flexing or stretching. Moreover, the conjugating member is restored to its original shape after joining to the groove. At this time, the thickness of the conjugating member is set to be roughly the same as the depth of the groove, so the outside surface of the connection and the outside surface of the conjugating member are connected so as to be positioned upon the same surface, and thus the medical tube is easily fitted to the connector and conjugating member.

Note that the stipulation that the "thickness of the conjugating member is set to be roughly the same as the depth of the groove" is not limited to the identical case but rather it includes values that are close, so it is adequate for no obvious step to arise at the boundary between the outside surface of the connector and the outside surface of the conjugating member. In addition, it is possible to provide a slit in the connecting material that extends in the axial direction, so that this slit can be opened and closed. Thereby, the conjugating member may be fitted to the connector in the state in which the slit is open, and then, after fitting to the connector, the conjugating member can be returned to its original cylindrical shape.

In addition, the structure for connecting between a medical needle and medical tubing according to the present invention is further **characterized in that:** said connector is formed in a columnar shape; said joint is constituted with a flange formed on the outside surface of said connector so as to protrude outward thereupon; and said conjugating member is constituted with a cylindrical body provided with a groove that is able to join to said joint. Thereby, movement of the conjugating member with respect to the medical needle in the direction of the axis of the medical tube can be effectively prevented, so it is possible to prevent the conjugating member and medical tubing from coming off of the medical needle. Note that the connector in this case may be formed in the shape of a circular column, triangular column, square column or other polygonal columns or other columnar shapes.

In addition, the structure for connecting between a medical needle and medical tubing according to the present invention is further **characterized in that:** said conjugating member is constituted with a plurality of members that are divided in the direction around the axis of cylindrical body. Thereby, the individual members constituting the conjugating member may be sequentially disposed around the connector, thus simplifying the work of fitting the conjugating member to the connector.

In addition, the structure for connecting between a medical needle and medical tubing according to the present invention is further **characterized in that:** a portion not covered with said conjugating member is provided at the base end of said connector, and a gap is provided between the base end of said connector and said medical tubing. Thereby, it is possible to prevent the outer peripheral edge of the opening of the medical tubing from floating up on the outer periphery side. The gap in this case should be of a depth that can prevent the end of the medical tubing from being pushed open by the connector toward the outer peripheral side. [Best Mode of Working the Invention]

### (Preferred Embodiment 1)

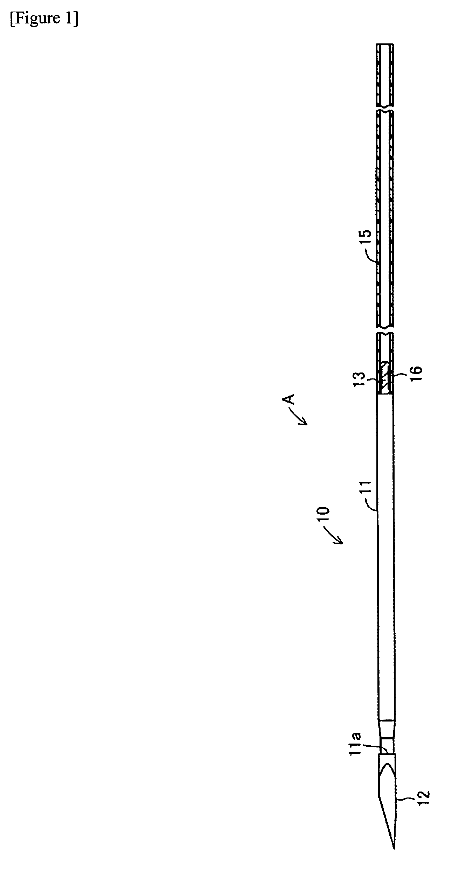

Here follows a description of the structure for connecting between a medical needle and medical tubing as a first embodiment of the present invention made with reference to drawings. FIG. 1 shows the connecting structure A according to this embodiment, where this connecting structure A comprises a medical needle 10, medical tubing 15 and an inner tube 16 serving as the conjugating member according to the present invention. Medical needle 10 comprises a cylindrical needle body 11 with a small diameter, a puncturing portion 12 formed at the tip of the needle body 11 and a connector 13 formed at the rear end of the needle body 11. In addition, a narrow portion is formed at the boundary between the needle body 11 and the puncturing portion 12, where the diameter is gradually made smaller when heading from the needle body 11 toward the puncturing portion 12 and thereafter kept at the same diameter, and a joining step 11a is formed at the front end thereof.

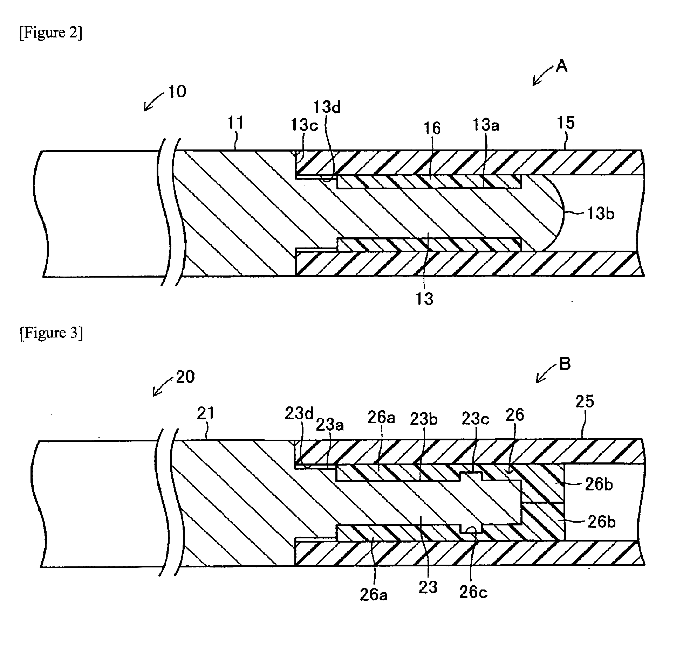

As shown in FIG. 2, the connector 13 is provided in order to connect the medical tubing 15 to the medical needle 10, being formed roughly in the shape of a cylinder with a diameter smaller than that of the needle body 11. A wide joining groove 13a constituting the joint according to the present invention is formed in the peripheral surface of this connector 13, in portions excluding the portions at either end in the axial direction, and the rear-end surface 13b of the connector 13 is formed in the shape of a smooth convex curved surface. In addition, a step 13c comprising a surface that is perpendicular to the axial direction of the medical needle 10 is formed between the needle body 11 and the connector 13, and the diameter of the portion between the joining groove 13a and the step 13c on the outer peripheral surface of the connector 13 is set to be somewhat smaller than the diameter of the rear-end portion of the connector 13 (the portion towards the right in FIG. 2).

The inner tube 16 constitutes a cylinder that is provided with flexibility and elasticity, and can be fit within the joining groove 13a of the connector 13 by pushing forward with its opening positioned at the rear-end surface 13b of the connector 13. In this case, the rear-end surface 13b is formed in the shape of a smooth convex curved surface, so the work of fitting the inner tube 16 within the joining groove 13a can be performed smoothly. Moreover, when the inner tube 16 is fitted within the joining groove 13a, the outer peripheral surface of the inner tube 16 and the outer peripheral surface of the rear-end portion of the connector 13 form a continuous curved surface with no difference in height. In this case, a small amount of difference in height may occur as long as it is not a noticeable step. In addition, a slight difference in height is formed between the outer peripheral surface of the inner tube 16 and the outer peripheral surface of the base-end portion of the connector 13. This inner tube 16 may be made of polyvinyl chloride or polyurethane.

The medical tubing 15 may be made of polyvinyl chloride or polyurethane, having an inside diameter such that it can cover the outside of the inner tube 16 and connector 13 which forms a cylinder with the inner tube 16 inserted within the joining groove 13a. This medical tubing 15 may be fit to the outside of the connector 13 with the inner tube 16 fit on, by pushing forward with its opening positioned at the rear-end surface 13b of the connector 13, and may be secured to the inner tube 16 by adhesive or welding.

In this case also, the rear-end surface 13b is formed in the shape of a smooth convex curved surface, so the work of fitting the medical tubing 15 to the connector 13 can be performed smoothly. When the medical tubing 15 is fitted to the connector 13, a gap 13d is formed between the medical tubing 15 and the outside peripheral surface of the base-end portion of the connector 13, while the medical tubing 15 is in close contact with the outside peripheral surface of the rear-end portion of the connector 13. In addition, the end surface of the medical tubing 15 is in contact with the step 13c of the medical needle 10 in the state such that the outside peripheral edge is prevented from protruding from the outside peripheral edge toward the outside.

When the medical tubing 15 is to be secured to the inner tube 16 by adhesive, examples of the adhesives that can be used include: ultraviolet-curing adhesives, visible light-curing adhesives, instant adhesives, or the like. In addition, when the medical tubing 15 is to be secured to the inner tube 16 by welding, it is possible to use the solvent welding method or the heat welding method. When solvent welding is to be used, it is possible to use cyclohexane, tetrahydrofuran or other solvents. When an adhesive or solvent is to be used, the inner tube 16 and the medical tubing 15 are assembled in the state with the outside peripheral surface of the inner tube 16 or the inside peripheral surface of the medical tubing 15 coated with adhesive or solvent.

In the case of using the heat welding method, in the state with the inner tube 16 and medical tubing 15 assembled, the medical needle 10 is heated to a temperature of 200°C, for example, using a stipulated heating apparatus and heat-shrinking tube. In this case, first the connecting portion of the medical tubing 15 and inner tube 16 is covered with a heat-shrinking tube. Then, the portion of the medical tubing 15 and inner tube 16 covered with the heat-shrinking tube is heated. Next, the connecting portion of the medical tubing 15 and inner tube 16 is melted by heating and unitized and then that portion is cooled. Thereby, the welding of the medical tubing 15 to the inner tube 16 is complete. Then, the heat-shrinking tube is removed from the welded medical tubing 15 and inner tube 16. The connecting structure A shown in FIG. 1 is thus obtained by these methods.

With this structure, when the medical tubing 15 is to be inserted into a wound (not shown) of a patient, first the medical needle 10 with the connecting structure A is inserted from an incision in the body of the patient toward the wound, and then the puncturing portion 12 at the tip is brought outside the body from the stipulated portion. At the time of this puncture, if necessary, it is possible to bend appropriate portions of the needle body 11 so that the medical needle 10 can puncture the appropriate position of the wound. In this case, the surface of the connector between the medical needle 10 and the medical tubing 15 comprises a smooth curved surface with no unevenness, so the resistance at the time of piercing is reduced so smooth operation becomes possible. In addition, a gap 13d is formed between the medical tubing 15 and the outer peripheral surface of the base-end portion of the connector 13, so the tip of the medical tubing 15 does not protrude toward the outside.

This prevents the tip of the medical tubing 15 from protruding toward the outside and interfering with the piercing. Moreover, once the puncturing portion 12 of the medical needle 10 has left the body, it is possible to attach a stipulated withdrawal tool (not shown) to the joining step 11a positioned at the rear of the puncturing portion 12, and thus manually hold the withdrawal tool and pull to draw the medical needle 10 outside of the body. Once the tip end of the medical tubing 15 comes out of the body, and the rear end of the medical tubing 15 is positioned at the wound, the pulling operation is halted and scissors are used to cut off the medical needle 10 and the connector-side portion of the medical tubing 15. Next, the cut portion of the medical tubing 15 is connected to a stipulated suction pump (not shown). The operation of the suction pump sucks out the blood and other exudates that collect in the wound via the medical tubing 15.

In this manner, with the connecting structure A according to this preferred embodiment, a cylindrical inner tube 16 is fitted to the joining groove 13a of the connector 13, and thus the connector 13 and inner tube 16 form a cylindrical shape. Then, the cylindrically formed outer peripheral surface of the connector 13 and inner tube 16 are covered with one end of the medical tubing 15, and the inner tube 16 is secured to the medical tubing 15 by welding or adhesive. Thereby, there is no unevenness in the outer peripheral surface of the connector 13 and inner tube 16 at their position in the interior of the medical tubing 15, so no unevenness occurs in the surface of the medical tubing 15 either. As a result, the resistance is reduced at the time that the medical needle 10 is pierced through the body of the patient, and smooth handling is possible.

In addition, a gap 13d is formed between the medical tubing 15 and the outside peripheral surface of the base-end portion of the connector 13, so the tip of the medical tubing 15 does not protrude from the connector 13 toward the outside. Thereby, the piercing by the medical needle 10 can be performed even more smoothly. In addition, the connecting structure A according to this preferred embodiment has the joint of the connector 13 constituting a joining groove 13a and the joining member constituting a cylindrical inner tube 16 which is joined to this joining groove 13a, and thus it is possible to obtain a connecting structure A with a simple constitution where no unevenness occurs in the connecting portion. Moreover, since it is possible to select the material to constitute the inner tube 16 as one that can be readily adhered or welded to the medical tubing 15, there is no need to use special materials.

### (Preferred Embodiment 2)

FIG. 2 shows the connecting structure B according to a second preferred embodiment of the present invention. With this connecting structure B, connector 23 of medical needle 20 is formed in the shape of a stepped rod with a circular cross section of a diameter smaller than that of the needle body 21. To wit, this connector 23 comprises a cylindrical large-diameter portion 23a positioned at the base-end side and having a short length in the axial direction, and a cylindrical small-diameter portion 23b that is concentric to the large-diameter portion 23a, is positioned at the rear-end side of the large-diameter portion 23a, has a longer length in the axial direction and has a diameter smaller than that of the large-diameter portion 23a. Moreover, a flange-shaped joint 23c that protrudes toward the outside along the circumference of the rear-end side portion of the small-diameter portion 23b (the portion toward the right side of FIG. 2). This joint 23c protrudes from the outer peripheral surface of the small-diameter portion 23b by a distance that somewhat greater than half of the distance between the rear edge of the outer peripheral surface of the large-diameter portion 23a and the front edge of the outer peripheral surface of the small-diameter portion 23b. In addition, the rear-end surface of the small-diameter portion 23b is formed as a plane.

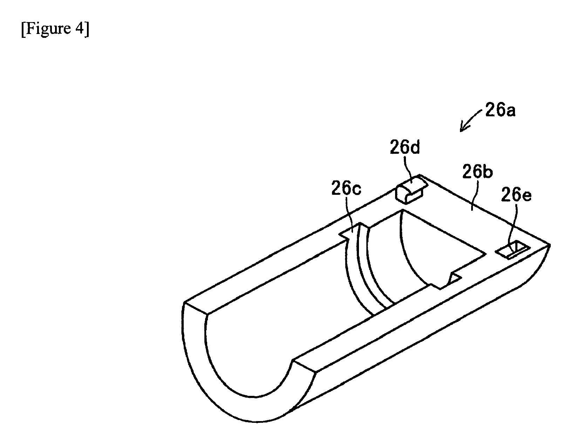

Moreover, as the conjugating member according to the present invention, a conjugating molded form 26 is attached to the small-diameter portion 23b over its outer peripheral surface and rear-end surface. This conjugating molded form 26 comprises two split members 26a of the same shape that are made of hard materials, so by assembling the two split members 26a, a cylindrical form with its rear end portion closed is formed. As shown in FIG. 4, each of the split members 26a is formed in the shape of a semicylindrical gutter with one end closed by a semicircular plate-shaped wall 26b, and a joint groove 26c is formed, on the inner peripheral surface toward the side of the wall 26b from the center in the axial direction, in the peripheral direction so as to be able to join to one half of the joint 23c along its periphery. Moreover, a joining prong 26d is formed on one side of the meeting surface (the upper surface in FIG. 4) of the wall 26b, and a joining hole 26e is formed on the other side of the meeting surface of the wall 26b so that it is able to join to the joining prong 26d of the other of the split members 26a.

Thus, when the two split members 26a are fitted to the outer periphery of the small-diameter portion 23b of the connector 23 with the two joint grooves 26c each joined to the joint 23c and also with the joining prongs 26d and joining holes 26e joined to each other, the small-diameter portion 23b and joint 23c are covered by the two split members 26a. As a result, only the outer peripheral surface and end surface of the conjugating molded form 26 made up of two split members 26a appears at the rear-end portion of the medical needle 20. The thickness of the cylindrical portion of the conjugating molded form 26 is set to be roughly twice the length of protrusion of the joint 23c from the outer peripheral surface of the small-diameter portion 23b, and for this reason, a slight step is formed between the outer peripheral surface of the conjugating molded form 26 and the outer peripheral surface of the large-diameter portion 23a of the connector 23. Examples of the material used to make this conjugating molded form 26 include: polyvinyl chloride, polyurethane, polycarbonate, acrylonitrile-butadiene-styrene copolymer resin, etc.

The medical tubing 25 is secured to the conjugating molded form 26 by adhesive or welding, and a gap 23d is formed between the medical tubing 25 and the outside peripheral surface of the large-diameter portion 23a of the connector 13 [*sic*]. In addition, the end surface of the medical tubing 25 is in contact with the step between the needle body 21 and the large-diameter portion 23a in the state such that the outside peripheral edge is prevented from protruding from the outside peripheral edge of the needle body 21 toward the outside. The constitution of other portions of this connecting structure B are identical to those of the aforementioned connecting structure A. In addition, the method of using the medical needle 20 and medical tubing 25 provided with this connecting structure B is identical to the method of using the aforementioned Preferred Embodiment 1.

By means of this constitution, movement of the conjugating molded form 26 and medical tubing 25 with respect to the medical needle 20 in the direction of the axis of the medical tube can be effectively prevented, so it is possible to prevent the conjugating molded form 26 and medical tubing 25 from coming off of the medical needle 20. In addition, the conjugating molded form 26 consists of two split members 26a, so the conjugating molded form 26 can be easily fit to the small-diameter portion 23b. Moreover, with this connecting structure B, the joint 23c of the connector 23 is embedded within the joint groove 26c of the conjugating molded form 26 to a depth of roughly ½ of the wall thickness of the conjugating molded form 26, so the conjugating molded form 26 can be fit to the connector 23 while maintaining the strength of the conjugating molded form 26. the other meritorious effects of connecting structure B are the same as the meritorious effects of the aforementioned connecting structure A.

In addition, the present invention is in no way limited to the aforementioned preferred embodiments and may be worked with appropriate modifications. For example, in the aforementioned Preferred Embodiment 2, the cross-sectional shape of the connector 23 is set to be round, but the cross-sectional shape of the connector 23 need not be round, but rather it may also be triangular, square or another polygonal shape. In addition, with the aforementioned Preferred Embodiment 2, the conjugating molded form 26 takes the form of a cylinder with the end closed, but this conjugating molded form 26 may also constitute two or more split members.

In addition, while the inner tube 16 comprises a heat-shrinking tube that is shrunk by heating and then cooling, it may also be secured to the connector 13 or 23 by being fitted to the connector 13 or 23 and then heated and cooled. In this case, it is preferable that the inner tube 16 and medical tubing 15 be made of polyvinyl chloride. Moreover, it is also possible to provide a slit extending in the axial direction of the inner tube 16, and permit this slit to open and close. In this case, the conjugating member may be fit to the connector in the state with the slit open and after fitting to the connector, the conjugating member is restored to its original cylindrical shape.

### [Brief Description of the Drawings]

[FIG. 1] This is a partial-cutaway side view showing the connecting structure according to Preferred Embodiment 1 of the present invention.
[FIG. 2] This is a cross section of the main portion of the connecting structure.
[FIG. 3] This is a cross section of the main portion of the connecting structure according to Preferred Embodiment 2 of the present invention.
[FIG. 4] This is a perspective view showing the split member.

### [Explanation of Symbols]

10, 20 ... medical needle, 13, 23 ... connector, 13a ... joining groove, 13d, 23d ... gap, 15, 25 ... medical tubing, 16 ... inner tube, 23c ... joint, 26 ... conjugating molded form, 26c ... joint groove, 26a ... split members, A, B ... connecting structure.

## Claims

1. A structure for connecting between a medical needle and medical tubing **characterized in** comprising:
a medical needle wherein a connector provided with a joint is formed upon its rear end;
a conjugating member that is joined to said joint in the state such that it covers at least part of said connector, and is formed in a cylindrical shape at said connector when it covers at least part of said connector; and
medical tubing that is connected to said medical needle by covering a cylindrical body made up of said connector and said conjugating member in the state in which the inside surface at one end is secured by welding or adhesion to the outside surface of said conjugating member.

2. The structure for connecting between a medical needle and medical tubing according to claim 1, wherein:
said connector is formed in a cylindrical shape; said joint is constituted with a groove formed on the outside surface of said connector along the circumference thereof; said conjugating member is constituted with a elastic cylindrical body that joins to said groove; and also, the thickness of said conjugating member is set to be approximately the same as the depth of said groove.

3. The structure for connecting between a medical needle and medical tubing according to claim 1, wherein:
said connector is formed in a columnar shape; said joint is constituted with a flange formed on the outside surface of said connector so as to protrude outward thereupon; and said conjugating member is constituted with a cylindrical body provided with a groove that is able to join to said joint.

4. The structure for connecting between a medical needle and medical tubing according to claim 2 or claim 3, wherein: said conjugating member is constituted with a plurality of members that are divided in the direction around the axis of cylindrical body.

5. The structure for connecting between a medical needle and medical tubing according to any of claims 1-4 wherein: a portion not covered with said conjugating member is provided at the base end of said connector, and a gap is provided between the base end of said connector and said medical tubing.
